(19) Europäisches Patentamt / European Patent Office / Office européen des brevets

(11) **EP 4 474 411 A1**

(12) **EUROPEAN PATENT APPLICATION**
published in accordance with Art. 153(4) EPC

(43) Date of publication:
**11.12.2024 Bulletin 2024/50**

(21) Application number: 22924970.1

(22) Date of filing: **30.11.2022**

(51) International Patent Classification (IPC):
$C08J\ 7/06$ (2006.01)    $A61K\ 8/73$ (2006.01)
$A61Q\ 1/02$ (2006.01)    $C08J\ 3/00$ (2006.01)

(52) Cooperative Patent Classification (CPC):
**A61K 8/73; A61Q 1/02; C08J 3/00; C08J 3/12;
C08J 7/06**

(86) International application number:
**PCT/JP2022/044145**

(87) International publication number:
**WO 2023/149065 (10.08.2023 Gazette 2023/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.02.2022 JP 2022014392**

(71) Applicant: **Dainichiseika Color & Chemicals Mfg.
Co., Ltd.
Chuo-ku
Tokyo 103-8383 (JP)**

(72) Inventor: **SUZUKI Shiho
Tokyo 103-8383 (JP)**

(74) Representative: **Wächtershäuser & Hartz
Patentanwaltspartnerschaft mbB
Weinstraße 8
80333 München (DE)**

(54) **WATER REPELLENT CELLULOSE BEADS, MANUFACTURING METHOD THEREFOR, AND COSMETIC**

(57) There are provided: water-repellent cellulose beads that are excellent in water repellency, easily spreadable on the skin without leaving a white residue, and unlikely to cause squeaky impression, and exhibit a smooth tactile impression; and a method for producing the same. The water-repellent cellulose is obtained by surface-treating cellulose beads having, for example, a cumulative 50% particle size on a volume basis of 3 to 15 µm and a degree of sphericity of 0.6 to 1.0 with hydrogenated lecithin containing 40 to 85% by mass of phosphatidylcholine. The method for producing water-repellent cellulose beads includes: mixing cellulose beads, hydrogenated lecithin, and water to obtain a mixed liquid; and subjecting the mixed liquid to filtration.

EP 4 474 411 A1

# EP 4 474 411 A1

## Description

### Technical Field

The present invention relates to water-repellent cellulose beads and a method for producing the same, and a cosmetic.

### Background Art

Particulate cellulose (cellulose beads) is known as a material with high safety and soft tactile impression, and is widely used for various products such as cosmetics. With the diversification of the needs of cosmetics and the high functionalization of cosmetics, functions that have never been required so far have also been required for materials for cosmetics.

It cannot be said that cellulose beads are materials having particularly favorable water repellency. For this reason, when a cosmetic in which cellulose beads are blended is used, makeup has been likely to come off due to perspiration or sebum in some cases. In addition, emulsion-type cosmetics prepared by blending cellulose beads may be likely to cause defects, for example, such that separation occurs to emulsion-type cosmetics prepared by blending cellulose beads after a long period of time, and therefore measures to solve these defects have been required.

In order to solve the defects as described above, there have been various proposals on enhancing the water repellency by surface-treating cellulose beads. For example, there is proposed a microcrystalline cellulose powder obtained by surface-treating crystalline cellulose with hydrogenated lecithin under the presence of a metal salt (Patent Literature 1). In addition, there is proposed a cellulose powder obtained by subjecting a cellulose powder to dry pulverization treatment together with hydrogenated lecithin (Patent Literature 2). Further, there is proposed surface-treating resin beads formed with a resin mainly composed of cellulose with a treatment agent such as oil and fats (Patent Literature 3).

### Citation List

### Patent Literature

Patent Literature 1: Japanese Patent Laid-Open No. 2003-146829
Patent Literature 2: International Publication No. WO 2011/105535
Patent Literature 3: Japanese Patent No. 6872068

### Summary of Invention

### Technical Problem

A certain extent of water repellency is imparted to the cellulose powders and the like proposed in Patent Literatures 1 to 3 by surface treatment. However, it cannot necessarily be said that the water repellency of these cellulose powders is sufficient, and there is further room for improvements. In addition, conventional surface-treated cellulose powders and the like are likely to leave a white residue when they are applied on the skin; and are likely to cause squeaky impression when they are spread on the skin, and therefore the conventional surface-treated cellulose powders and the like do not necessarily exhibit a smooth tactile impression.

The present invention has been completed in view of such problems of the conventional techniques, and an object of the present invention is to provide water-repellent cellulose beads that are excellent in water repellency, easily spreadable on the skin without leaving a white residue, and unlikely to cause squeaky impression, and exhibit a smooth tactile impression. In addition, another object of the present invention is to provide: a method for producing the above-described water-repellent cellulose beads; and a cosmetic using the above-described water-repellent cellulose beads.

### Solution to Problem

That is, according to the present invention, there are provided water-repellent cellulose beads, described below.

[1] Water-repellent cellulose beads obtained by surface-treating cellulose beads with hydrogenated lecithin containing 40 to 85% by mass of phosphatidylcholine.

2

[2] The water-repellent cellulose beads according to [1], wherein the hydrogenated lecithin has an acid value of 5 to 25 mgKOH/g and an iodine value of 0.1 to 2 $I_2$ g/100 g.

[3] The water-repellent cellulose beads according to [1] or [2], wherein the cellulose beads have a cumulative 50% particle size on a volume basis of 3 to 15 $\mu$m and a degree of sphericity of 0.6 to 1.0.

In addition, according to the present invention, there is provided a method for producing water-repellent cellulose beads, described below.

[4] A method for producing water-repellent cellulose beads, the method including: mixing cellulose beads, hydrogenated lecithin comprising 40 to 85% by mass of phosphatidylcholine, and water to obtain a mixed liquid; and subjecting the mixed liquid to filtration.

[5] The method for producing water-repellent cellulose beads according to [4], wherein the amount of the hydrogenated lecithin based on 100 parts by mass of the cellulose beads is 0.1 to 5 parts by mass.

[6] The method for producing water-repellent cellulose beads according to [4] or [5], wherein the mixed liquid is obtained as the one heated to 50 to 90°C.

[7] The method for producing water-repellent cellulose beads according to any one of [4] to [6], wherein the mixed liquid whose temperature is adjusted to 0 to 40°C is subjected to filtration.

Further, according to the present invention, there is provided a cosmetic, described below.

[8] A cosmetic containing the water-repellent cellulose beads according to any one of [1] to [3].

**Advantageous Effects of Invention**

According to the present invention, it is possible to provide water-repellent cellulose beads that are excellent in water repellency, easily spreadable on the skin without leaving a white residue, and unlikely to cause squeaky impression, and exhibit a smooth tactile impression. In addition, according to the present invention, it is possible to provide a method for producing the water-repellent cellulose beads and a cosmetic using the water-repellent cellulose beads.

**Description of Embodiments**

<Water-Repellent Cellulose Beads>

Hereinafter, embodiments of the present invention will be described, but the present invention is not limited to the following embodiments. Water-repellent cellulose beads of the present invention are obtained by surface-treating cellulose beads with hydrogenated lecithin containing 40 to 85% by mass of phosphatidylcholine. Hereinafter, the water-repellent cellulose beads of the present invention are also simply referred to as "water-repellent beads" or "beads."

(Cellulose Beads)

The water-repellent cellulose beads are obtained by surface-treating cellulose beads which is used as a raw material with hydrogenated lecithin. When the cellulose beads are subjected to surface treatment with the hydrogenated lecithin which functions as a surface treatment agent, it is inferred that a certain type of coating layer is formed by adhesion, supporting, bonding, or the like of the hydrogenated lecithin onto at least part of the surfaces of the cellulose beads. However, it is substantially difficult or impossible to analyze and check the adhesion state of the hydrogenated lecithin and the states (range, adhesion amount, thickness, and the like) of the coating layer formed due to adhesion or the like of the hydrogenated lecithin to the cellulose beads.

The cellulose beads are resin beads formed with a resin mainly composed of cellulose, preferably formed with cellulose. Note that in the cellulose, some of the hydroxy groups in the cellulose molecule may form ester bonds. In other words, a resin for forming the cellulose beads contains cellulose as the main ingredient and may further contain a slight amount of a cellulose ester, such as cellulose acetate and cellulose acetate propionate.

The shape of the cellulose beads is preferably a spherical shape, such as an approximately spherical shape, a spherical shape, and perfectly spherical shape. By surface-treating the cellulose beads having a spherical shape, water-repellent beads that are easily spreadable on the skin, unlikely to cause squeaky impression, and exhibit a smooth tactile impression can be prepared.

The cumulative 50% particle size (median size; $D_{50}$) on a volume basis of the cellulose beads is preferably 3 to 15 $\mu$m, more preferably 5 to 12 $\mu$m. By surface-treating the cellulose beads having $D_{50}$ within the above-described range, the slipperiness and soft-focus performance, which are required in resin beads to be blended in a cosmetic, can be exhibited more effectively.

Note that even when the cellulose beads are subjected to the surface treatment, the particle size is hardly increased and is not substantially changed. For this reason, the particle size of the cellulose beads before the surface treatment which are used as a raw material is substantially the same as the particle size of the water-repellent cellulose beads after the surface treatment. Accordingly, the cumulative 50% particle size ($D_{50}$) on a volume basis of the water-repellent cellulose beads as well as the above-described cumulative 50% particle size ($D_{50}$) on a volume basis of the cellulose beads is also preferably 3 to 15 $\mu$m, more preferably 5 to 12 $\mu$m.

The degree of sphericity of the cellulose beads is preferably 0.6 to 1.0, more preferably 0.7 to 1.0, particularly preferably 0.75 to 1.0, most preferably 0.8 to 1.0. By surface-treating the cellulose beads having a degree of sphericity within the above-described range, spreadability on the skin and excellent tactile impression, which are required in resin beads to be blended in a cosmetic, can be exhibited more effectively.

Note that even when the cellulose beads are subjected to the surface treatment, the degree of sphericity is not substantially changed. For this reason, the degree of sphericity of the cellulose beads before the surface treatment which are used as a raw material is substantially the same as the degree of sphericity of the water-repellent cellulose beads after the surface treatment. Accordingly, the degree of sphericity of the water-repellent cellulose beads as well as the above-described degree of sphericity of the cellulose beads is also preferably 0.6 to 1.0, more preferably 0.7 to 1.0, particularly preferably 0.75 to 1.0, most preferably 0.8 to 1.0.

The degree of sphericity, which is an index of whether the beads have a perfectly spherical shape or not, can be measured and calculated according to the procedure described below. Firstly, a SEM image of the beads, taken with a scanning electron microscope (SEM), is subjected to image analysis to calculate the degree of circularity C for each resin bead from the following formula (1). Then, the arithmetic average value of the degrees of circularity C for 10 or more resin beads arbitrarily selected is defined as the degree of sphericity.

$$C \ = \ (4\pi S_1)/(L^2) \ \cdots \ (1)$$

In the formula (1), $S_1$ represents the area (projected area) of each bead in the image, and L represents the length of the outer peripheral part of the bead in the image. As the value of the degree of circularity C is closer to 1, the shape of a particle is closer to a perfect sphere.

Commercially available cellulose beads may be used as the cellulose beads, or cellulose beads produced according to any of the conventionally known methods may be used. Examples of the methods for producing cellulose beads include the method described in Japanese Patent No. 6872068 (Patent Literature 3), described above.

(Hydrogenated Lecithin)

By surface-treating the cellulose beads with hydrogenated lecithin, the water-repellent cellulose beads can be obtained. Specifically, hydrogenated lecithin is an ingredient that functions as a surface treatment agent for the cellulose beads.

The phosphatidylcholine content (hereinafter, also referred to as "PC value" for convenience) of hydrogenated lecithin is 40 to 85% by mass, preferably 45 to 80% by mass, more preferably 50 to 78% by mass. By surface-treating the cellulose beads with hydrogenated lecithin having a PC value within the above-described range, the water-repellent cellulose beads that are excellent in water repellency, easily spreadable on the skin without leaving a white residue, and unlikely to cause squeaky impression, and exhibit a smooth tactile impression can be prepared. Note that the phosphatidylcholine content (PC value) of hydrogenated lecithin can be analyzed and measured according to the methods such as , for example, "Thin Layer Chromatograph (TLC Method)," "High Performance Liquid Chromatograph (HPLC Method)," and the like in "Testing/Analyzing Method for Conjugated Lipid," described in "Oleo Science, Vol. 1, No.6(2001), p. 63 to 71."

The acid value of the hydrogenated lecithin is preferably 5 to 25 mgKOH/g, more preferably 8 to 20 mgKOH/g. By surface-treating the cellulose beads with the hydrogenated lecithin having an acid value within the above-described range, water-repellent cellulose beads more excellent in water repellency can be prepared.

The iodine value of the hydrogenated lecithin is preferably 0.1 to 2 $I_2$ g/100 g, more preferably 0.3 to 1.7 $I_2$ g/100 g. In the hydrogenated lecithin, at least part of the unsaturated double bonds in the lecithin molecule is reduced by hydrogenation. The iodine value is a physical property value corresponding to the content of the unsaturated double bond in the hydrogenated lecithin molecule, and hydrogenated lecithin having an iodine value of less than 0.1 $I_2$ g/100 g is substantially hardly available. On the other hand, when the iodine value of hydrogenated lecithin is more than 2 $I_2$ g/100 g, light stability or the like of resultant water-repellent cellulose beads may be somewhat lowered in some cases.

<Method for Producing Water-Repellent Cellulose Beads>

The above-described water-repellent cellulose beads can be produced by a production method described below. Specifically, the method for producing the water-repellent cellulose beads of the present invention includes a step (step (1)) of mixing cellulose beads, hydrogenated lecithin containing 40 to 85% by mass of phosphatidylcholine, and water to obtain a mixed liquid; and a step (step (2)) of subjecting the resultant mixed liquid to filtration.

(Step (1))

In the step (1), the cellulose beads, the hydrogenated lecithin, and water are mixed to obtain a mixed liquid. Specifically, the cellulose beads are subjected to surface treatment with the hydrogenated lecithin under a wet condition. In contrast, when the cellulose beads are treated with the hydrogenated lecithin under a dry condition (in the absence of water), it is difficult to obtain water-repellent cellulose beads having sufficiently enhanced water repellency.

The amount of the hydrogenated lecithin based on 100 parts by mass of the cellulose beads is preferably set to 0.1 to 5 parts by mass, more preferably 0.2 to 4 parts by mass, particularly preferably 0.25 to 3 parts by mass. When the amount of the hydrogenated lecithin based on 100 parts by mass of the cellulose beads is less than 0.1 parts by mass, it may be difficult to enhance the water repellency of resultant water-repellent cellulose beads sufficiently in some cases. On the other hand, when the amount of the hydrogenated lecithin based on 100 parts by mass of the cellulose beads is more than 5 parts by mass, the tactile impression of resultant water-repellent cellulose beads may be somewhat heavy in some cases, and the spreadability on the skin may be somewhat lowered in some cases.

The cellulose beads, the hydrogenated lecithin, and water, when mixed, are preferably heated to an appropriate temperature. Specifically, the mixed liquid heated to 50 to 90°C is preferably obtained, and the mixed liquid heated to 60 to 80°C is more preferably obtained. Heating the mixed liquid makes it possible to treat the surfaces of the cellulose beads more efficiently with the hydrogenated lecithin and makes it possible to obtain water-repellent cellulose beads having further improved properties such as water repellency.

The mixed liquid can be prepared by, for example, mixing the cellulose beads, the hydrogenated lecithin, and water all together at a predetermined temperature. In addition, the mixed liquid may be prepared by mixing a cellulose dispersion having a predetermined temperature and a hydrogenated lecithin dispersion having a predetermined temperature, wherein the cellulose beads dispersion is obtained by mixing the cellulose beads and water, and the hydrogenated lecithin dispersion is obtained by mixing the hydrogenated lecithin and water.

In the mixed liquid, the content of a metal salt of aluminum, magnesium, potassium, zinc, zirconium, titanium, or the like is preferably less than 0.1 equivalent, more preferably 0.05 equivalent or less on a mass basis based on the amount of the hydrogenated lecithin. In addition, it is particularly preferable that the mixed liquid be substantially free from the metal salt. That the metal salt is excessively contained in the mixed liquid means that a certain amount of a metal is also contained in water-repellent cellulose beads obtained from the mixed liquid. When water-repellent cellulose beads excessively containing a metal are used, there is a tendency that the stability of resultant cosmetic is lowered.

(Step (2))

In the step (2), the mixed liquid prepared in the step (1) is subjected to filtration. Note that the mixed liquid whose temperature is adjusted to 0 to 40°C is preferably subjected to filtration, the mixed liquid whose temperature is adjusted to 10 to 30°C is more preferably subjected to filtration, and the mixed liquid whose temperature is adjusted to a temperature near room temperature (around 15 to 28°C) is particularly preferably subject to filtration. It is considered that by controlling the temperature of the mixed liquid within the above described range, at least part of hydrogenated lecithin is precipitated out. This makes it possible to treat the surfaces of the cellulose beads more efficiently with hydrogenated lecithin, and water-repellent cellulose beads having further improved properties such as water repellency can be obtained.

After the mixed liquid is subjected to filtration, rinsing, drying, pulverization, and the like are performed as necessary, and thus desired water-repellent cellulose beads can be obtained.

<Cosmetic>

The above-described water-repellent cellulose beads are excellent in water repellency, easily spreadable on the skin without leaving a white residue, and unlikely to cause squeaky impression, and exhibit a smooth tactile impression. Therefore, the use of the water-repellent cellulose beads as one of the raw materials makes it possible to prepare a cosmetic that is easily spreadable on the skin without leaving a white residue; that is unlikely to cause squeaky impression and exhibit a smooth tactile impression; and that is unlikely to, for example, be separated with time and is excellent in stability. That is, the cosmetic of the present invention contains the above-described water-repellent cellulose beads.

The amount of the water-repellent cellulose beads contained in the cosmetic is not particularly limited as long as it is appropriately designed according to the type and the like of the cosmetic. The content of the water-repellent cellulose beads in the cosmetic is usually 1 to 10% by mass, particularly 1 to 5% by mass, based on the total amount of the cosmetic.

The cosmetic contains ordinary raw materials which are used according to various types of cosmetics, such as loose powder, foundation, a sunblock, and all-in-one gel, as ingredients other than the above-described water-repellent cellulose beads. As the raw materials other than the water-repellent cellulose beads, various ingredients that appear in "International Cosmetic Ingredient Dictionary and Handbook" (13th edition, 2010) published by Personal Care Products Council can be used.

Examples of the raw material other than the water-repellent cellulose beads include fats and oils, waxes, higher fatty acids, higher alcohols, esters, hydrocarbons, silicone oils, surfactants, metal soap, polymeric compounds, humectants, extender pigments, organic colorants, inorganic colorants, white pigments, pearl pigments, ultraviolet absorbers, antioxidants, sequestrants, and water.

Examples of the cosmetic include loose powder, foundation, a sunblock, and all-in-one gel. Liquid foundation is classified into O/W emulsion type foundation, W/O emulsion type foundation, and the like. In the W/O emulsion type foundation, the outer phase is composed of oil, and therefore the impression after use is sticky in many cases. In contrast, by blending the above-described water-repellent cellulose beads, the sticky impression after use can be suppressed, and the emulsion stability is improved, which makes the W/O emulsion type foundation unlikely to, for example, be separated after a long period of time.

Examples

Hereinafter, the present invention will specifically be described based on Examples, but the present invention is not limited to these Examples. Note that "parts" and "%" in Examples and Comparative Examples are on a mass basis unless otherwise noted.

<Production of Cellulose Beads>

Two types of cellulose beads (cellulose beads 1 and 2) having a different cumulative 50% particle size ($D_{50}$ ($\mu$m)) on a volume basis and a different degree of sphericity were produced with reference to the description in "Example 1" and "Example 4" in Japanese Patent No. 6872068. Physical properties of the produced cellulose beads are shown in Table 1.

Table 1

| Cellulose beads | $D_{50}$ ($\mu$m) | Degree of sphericity |
|---|---|---|
| 1 | 8 | 0.96 |
| 2 | 10 | 0.97 |

<Providing Hydrogenated Lecithin>

Hydrogenated lecithin A to D (all commercially available products) of the types shown in Table 2 were provided. Table 2 shows PC values (phosphatidylcholine content; %), acid values (mgKOH/g), and iodine values ($I_2$ g/100g) of hydrogenated lecithin A to D. Note that all of the PC values of hydrogenated lecithin A to D are values described in the catalogs from the manufacturers (sales agents).

Table 2

| Hydrogenated lecithin | PC value (%) | Acid value (mgKOH/g) | Iodine value ($I_2$ g/100g) |
|---|---|---|---|
| A | 25-30 | 28 | 5.8 |
| B | 55-75 | 17 | 1.5 |
| C | 90 | - | 0.15 |
| D | 70 | 10.9 | 0.5 |

<Conditions for Producing Water-Repellent Cellulose Beads>

Conditions for producing water-repellent cellulose beads (production conditions 1 to 8) are described below.

(Production Condition 1)

The cellulose beads were put into 100 parts of water at 20°C, and then the resultant mixture was stirred at 4,000 rpm for 4 minutes using a homogenizer to obtain a cellulose beads dispersion. Further, the hydrogenated lecithin was added to 100 parts of water at 20°C, and then the resultant mixture was stirred at 4,000 rpm for 4 minutes using a homogenizer to obtain a hydrogenated lecithin dispersion. The hydrogenated lecithin dispersion was gradually added to the cellulose beads dispersion, and then the resultant mixture was stirred at 4,000 rpm for 4 minutes using a homogenizer.

The resultant mixture was subjected to filtration, and the crude product was rinsed with water and then dried at 100°C. The dried product was subjected to pulverization treatment for 10 seconds twice using a pot mill, and then the pulverized product was passed through a 300 Mesh sieve to obtain water-repellent cellulose beads.

(Production Condition 2)

The cellulose beads were put into 200 parts of water at 20°C. The resultant mixture was stirred at 4,000 rpm for 4 minutes using a homogenizer, and then the hydrogenated lecithin was added thereto. The resultant mixture was stirred at 4,000 rpm for 4 minutes using a homogenizer and was then subjected to filtration, and the crude product was rinsed with water. The product was dried at 100°C and then subjected to pulverization treatment for 10 seconds twice using a pot mill. Further, the pulverized product was passed through a 300 Mesh sieve to obtain water-repellent cellulose beads.

(Production Condition 3)

The cellulose beads were put into 100 parts of water at 70°C, and then the resultant mixture was stirred at 4,000 rpm for 4 minutes using a homogenizer to obtain a cellulose beads dispersion. Further, the hydrogenated lecithin was added to 100 parts of water at 70°C, and then the resultant mixture was stirred at 4,000 rpm for 4 minutes using a homogenizer to obtain a hydrogenated lecithin dispersion. The hydrogenated lecithin dispersion was gradually added to the cellulose beads dispersion, and then the resultant mixture was stirred at 4,000 rpm for 4 minutes using a homogenizer. The mixture was subjected to filtration in a state where the temperature of the mixture was kept at 70°C, and then the crude product was rinsed with water. After the rinsed product was dried at 100°C, the dried product was subjected to pulverization treatment for 10 seconds twice using a pot mill. Further, the pulverized product was passed through a 300 Mesh sieve to obtain water-repellent cellulose beads.

(Production Condition 4)

The cellulose beads were put into 200 parts of water at 70°C. The resultant mixture was stirred at 4,000 rpm for 4 minutes using a homogenizer, and then the hydrogenated lecithin was added thereto. The resultant mixture was stirred at 4,000 rpm for 4 minutes using a homogenizer. The mixture was subjected to filtration in a state where the temperature of the mixture was kept at 70°C, and then the crude product was rinsed with water. After the rinsed product was dried at 100°C, the dried product was subjected to pulverization treatment for 10 seconds twice using a pot mill. Further, the pulverized product was passed through a 300 Mesh sieve to obtain water-repellent cellulose beads.

(Production Condition 5)

The cellulose beads were put into 100 parts of water at 70°C, and then the resultant mixture was stirred at 4,000 rpm for 4 minutes using a homogenizer to obtain a cellulose beads dispersion. Further, the hydrogenated lecithin was added to 100 parts of water at 70°C, and then the resultant mixture was stirred at 4,000 rpm for 4 minutes using a homogenizer to obtain a hydrogenated lecithin dispersion. The hydrogenated lecithin dispersion was gradually added to the cellulose beads dispersion, and then the resultant mixture was stirred at 4,000 rpm for 4 minutes using a homogenizer. The mixture was cooled to room temperature (20°C) and subjected to filtration, and then the crude product was rinsed with water. After the rinsed product was dried at 100°C, the dried product was subjected to pulverization treatment for 10 seconds twice using a pot mill. Further, the pulverized product was passed through a 300 Mesh sieve to obtain water-repellent cellulose beads.

(Production Condition 6)

The cellulose beads were put into 200 parts of water at 70°C. The resultant mixture was stirred at 4,000 rpm for 4 minutes using a homogenizer, and then the hydrogenated lecithin was added thereto. The resultant mixture was stirred at 4,000 rpm for 4 minutes using a homogenizer. The mixture was cooled to room temperature (20°C) and subjected to filtration, and then the crude product was rinsed with water. After the rinsed product was dried at 100°C, the dried product was subjected to pulverization treatment for 10 seconds twice using a pot mill. Further, the pulverized product was passed through a 300 Mesh sieve to obtain water-repellent cellulose beads.

(Production Condition 7)

The cellulose beads were put into 100 parts of water at 70°C, and then the resultant mixture was stirred at 2,000 rpm for 4 minutes using a disper to obtain a cellulose beads dispersion. Further, the hydrogenated lecithin was added to 100 parts of water at 70°C, and then the resultant mixture was stirred at 2,000 rpm for 4 minutes using a disper to obtain a hydrogenated lecithin dispersion. The hydrogenated lecithin dispersion was gradually added to the cellulose beads dispersion, and then the resultant mixture was stirred at 2,000 rpm for 4 minutes using a disper. The mixture was cooled to room temperature (20°C) and subjected to filtration, and then the crude product was rinsed with water. After the rinsed product was dried at 100°C, the dried product was subjected to pulverization treatment for 10 seconds twice using a pot mill. Further, the pulverized product was passed through a 300 Mesh sieve to obtain water-repellent cellulose beads.

(Production Condition 8)

The cellulose beads and the hydrogenated lecithin were put into 200 parts of water at 20°C. The resultant mixture was stirred at 4,000 rpm for 4 minutes using a homogenizer. The mixture was heated to 70°C and then stirred at 4,000 rpm for 4 minutes using a homogenizer. The mixture was cooled to room temperature (20°C) and subjected to filtration, and then the crude product was rinsed with water. After the rinsed product was dried at 100°C, the dried product was subjected to pulverization treatment for 10 seconds twice using a pot mill. Further, the pulverized product was passed through a 300 Mesh sieve to obtain water-repellent cellulose beads.

<Production of Water-Repellent Cellulose Beads>

(Examples 1 to 13 and Comparative Examples 1 to 6)

Water-repellent cellulose beads were produced combining "Combination" and "Production condition" as shown in Table 4. Table 3 shows the details of the "Combination (Combinations 1 to 9)."

Table 3 (Units: parts)

| | | Combination 1 | Combination 2 | Combination 3 | Combination 4 | Combination 5 | Combination 6 | Combination 7 | Combination 8 | Combination 9 |
|---|---|---|---|---|---|---|---|---|---|---|
| Cellulose beads | 1 | 30 | 30 | 30 | 30 | 30 | 30 | 30 | 30 | |
| | 2 | | | | | | | | | 30 |
| Hydrogenated lecithin | A | 0.2 | | | | | | | | |
| | B | | 0.1 | 0.2 | 0.6 | | | | | 0.2 |
| | C | | | | | 0.2 | | | | |
| | D | | | | | | 0.2 | 0.6 | | |
| Water | | 200 | 200 | 200 | 200 | 200 | 200 | 200 | 200 | 200 |
| Amount (parts) of hydrogenated lecithin based on 100 parts of cellulose beads | | 0.7 | 0.3 | 0.7 | 2.0 | 0.7 | 0.7 | 2.0 | 0.0 | 0.7 |

<Evaluations of Beads>

(Water Repellency)

After 20 mL of ion-exchanged water was put into a 30 mL vial, 20 mg of the beads were put therein. After one day, the contents in the vial were observed and the water repellency of the beads was evaluated according to the evaluation criteria shown below. The results are shown in Table 4.

5: Beads are completely floating.
4: A slight number of beads are settled.
3: There are settled beads.
2: Most of the beads are settled
1: All the beads are settled.

(Suppression of White Residue)

Sensory evaluation on the suppression of white residue of the beads by a panel test of 10 test evaluators was performed. Specifically, the extent of white residue at the time when the beads were spread on the skin was totally decided and was graded on a scale of 1 to 5 according to the evaluation criteria shown below to calculate the average mark in the 10 test evaluators. The results are shown in Table 4.

5: Excellent
4: Above average
3: Average
2: Below average
1: Poor

(Spreadability)

Sensory evaluation on the spreadability of the beads by a panel test of 10 test evaluators was performed. Specifically, the spreadability of the beads on the skin was totally decided and was graded on a scale of 1 to 5 according to the evaluation criteria shown below to calculate the average mark in the 10 test evaluators. The results are shown in Table 4.

5: Excellent
4: Above average
3: Average
2: Below average
1: Poor

(Suppression of Squeaky Impression)

Sensory evaluation on the suppression of squeaky impression of the beads by a panel test of 10 test evaluators was performed. Specifically, "the sound like a squeak and the extent of how fingers are likely to be caught by the beads" at the time when the beads were spread on the skin were totally decided and were graded on a scale of 1 to 5 according to the evaluation criteria shown below to calculate the average mark in the 10 test evaluators. The results are shown in Table 4.

5: Excellent
4: Above average
3: Average
2: Below average
1: Poor

Table 4

| | | Combination | Production condition | Evaluations | | | |
|---|---|---|---|---|---|---|---|
| | | | | Water repellency | Suppression of white residue | Spreadability | Suppression of squeaky impression |
| Examples | 1 | 2 | (5) | 3 | 4.1 | 3.7 | 3.9 |
| | 2 | 3 | (1) | 4 | 3.4 | 3.2 | 3.2 |
| | 3 | 3 | (2) | 4 | 3.1 | 3 | 3.1 |
| | 4 | 3 | (3) | 5 | 4.2 | 3.6 | 4 |
| | 5 | 3 | (4) | 4 | 3.7 | 3.3 | 3.7 |
| | 6 | 3 | (5) | 5 | 4.8 | 4.3 | 4 .7 |
| | 7 | 3 | (6) | 5 | 4.4 | 3.9 | 4 . 4 |
| | 8 | 3 | (7) | 5 | 4.3 | 3.8 | 4.2 |
| | 9 | 3 | (8) | 5 | 4.5 | 4.1 | 4.6 |
| | 10 | 4 | (5) | 5 | 4.2 | 3.8 | 4 |
| | 11 | 6 | (5) | 5 | 4.4 | 3.9 | 4.3 |
| | 12 | 7 | (5) | 5 | 3.7 | 3.6 | 3.7 |
| | 13 | 9 | (5) | 5 | 4.5 | 3.8 | 4.1 |
| Comparative Examples | 1 | 1 | (1) | 1 | 1.5 | 1.7 | 1.7 |
| | 2 | 1 | (3) | 1 | 1.8 | 2 | 1.9 |
| | 3 | 1 | (5) | 2 | 2.1 | 2.2 | 2 |
| | 4 | 1 | (7) | 1 | 2 | 2.2 | 1.9 |
| | 5 | 5 | (5) | 1 | 1.8 | 2 | 2.3 |
| | 6 | 8 | (5) | 1 | 4.9 | 4.1 | 4.6 |

<Preparation of W/O Liquid Foundation>

The ingredients of A1 shown in Table 5 were mixed and heated to 60°C to be dissolved uniformly, and then the ingredients of A2 were added thereto and mixed uniformly. Into the resultant mixture of A1 and A2, the ingredients of C shown in Table 5 were added and dispersed, and thus a dispersion was obtained. Into the obtained dispersion, a mixture of the ingredients of B shown in Table 5 was gradually added under high-speed stirring and mixed uniformly, and thus W/O liquid foundation was obtained.

<Evaluations of W/O Liquid Foundation>

(Emulsified and Stirred State)

The emulsified and stirred state during preparing the W/O liquid foundation was "excellent."

(Emulsification Stability)

The prepared W/O liquid foundation was left standing under a condition of room temperature (20°C) and a condition of 40°C respectively. The W/O liquid foundation was observed visually every one month to evaluate the emulsification stability according to the evaluation criteria shown below. The results are shown in Table 5.

5: There is no change after the storage as compared to before the storage.
4: There is little change after the storage as compared to before the storage.
3: The W/O liquid foundation becomes somewhat non-uniform.
2: The W/O liquid foundation becomes non-uniform.

1: The W/O liquid foundation was separated into an oil phase and a water phase.

Table 5 (Units in upper rows: parts)

| | | | |
|---|---|---|---|
| A1 | UV absorber | | 5.50 |
| | Emollient | | 8.00 |
| A2 | Silicone oil | | 18.00 |
| | Emulsifying agent | | 4.20 |
| | Antiseptic | | 0.60 |
| | Defoamer | | 2.00 |
| C | Pigment | | 10.05 |
| | Water-repellent cellulose beads (Example 6) | | 3.00 |
| | Thickener | | 1.20 |
| B | Antiseptic | | 0.30 |
| | Polyhydric alcohol | | 9.00 |
| | Antistatic agent | | 0.20 |
| | Purified water | | 37.95 |
| Total | | | 100.00 |
| Evaluations | Emulsified and stirred state | | Excellent |
| | Emulsification stability | Room temperature — After 1 month | 5 |
| | | Room temperature — After 2 month | 5 |
| | | 40°C — After 1 month | 5 |
| | | 40°C — After 2 month | 5 |

**Industrial Applicability**

The water-repellent cellulose beads of the present invention are useful as a raw material to be used for cosmetics, such as W/O liquid foundation.

**Claims**

1. Water-repellent cellulose beads obtained by surface-treating cellulose beads with hydrogenated lecithin comprising 40 to 85% by mass of phosphatidylcholine, wherein
the amount of the hydrogenated lecithin based on 100 parts by mass of the cellulose beads is 0.1 to 5 parts by mass.

2. The water-repellent cellulose beads according to claim 1, wherein the hydrogenated lecithin has an acid value of 5 to 25 mgKOH/g and an iodine value of 0.1 to 2 $I_2$ g/100 g.

3. The water-repellent cellulose beads according to claim 1 or 2, wherein the cellulose beads have a cumulative 50% particle size on a volume basis of 3 to 15 $\mu$m and a degree of sphericity of 0.6 to 1.0.

4. A method for producing water-repellent cellulose beads, the method comprising:

a step of mixing cellulose beads, hydrogenated lecithin comprising 40 to 85% by mass of phosphatidylcholine, and water to obtain a mixed liquid; and
a step of subjecting the mixed liquid to filtration, wherein
the amount of the hydrogenated lecithin based on 100 parts by mass of the cellulose beads is 0.1 to 5 parts by mass.

5. The method for producing water-repellent cellulose beads according to claim 4, wherein the mixed liquid is obtained as the one heated to 50 to 90°C.

6. The method for producing water-repellent cellulose beads according to claim 4 or 5, wherein the mixed liquid whose temperature is adjusted to 0 to 40°C is subjected to filtration.

7. A cosmetic comprising the water-repellent cellulose beads according to any one of claims 1 to 3.

## INTERNATIONAL SEARCH REPORT

International application No.

**PCT/JP2022/044145**

### A. CLASSIFICATION OF SUBJECT MATTER

*C08J 7/06*(2006.01)i; *A61K 8/73*(2006.01)i; *A61Q 1/02*(2006.01)i; *C08J 3/00*(2006.01)i
FI:  C08J7/06 Z; A61K8/73; A61Q1/02; C08J3/00

According to International Patent Classification (IPC) or to both national classification and IPC

### B. FIELDS SEARCHED

Minimum documentation searched (classification system followed by classification symbols)

A61K8/00-8/99; A61Q1/00-90/00; C08J3/00-3/28; C08J7/04-7/06; C08J99/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | WO 2011/105535 A1 (OHKEN CO., LTD.) 01 September 2011 (2011-09-01) | 1-7 |
| A | JP 2010-77111 A (KOSE CORP) 08 April 2010 (2010-04-08) | 1-7 |
| A | WO 2020/188698 A1 (DAICEL CORPORATION) 24 September 2020 (2020-09-24) | 1-7 |
| A | WO 2020/137017 A1 (NIPPON FINE CHEMICAL CO) 02 July 2020 (2020-07-02) | 1-7 |
| A | JP 2014-28785 A (KOSE CORP) 13 February 2014 (2014-02-13) | 1-7 |
| A | JP 2007-291035 A (FANCL CORP) 08 November 2007 (2007-11-08) | 1-7 |
| A | JP 2020-109074 A (CHIFURE HOLDINGS CO LTD) 16 July 2020 (2020-07-16) | 1-7 |
| A | WO 2012/133018 A1 (OHKEN CO., LTD.) 04 October 2012 (2012-10-04) | 1-7 |
| A | WO 2010/067037 A1 (UNIVERSITE D'ANGERS) 17 June 2010 (2010-06-17) | 1-7 |

☐ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **08 February 2023** | **21 February 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2022/044145**

| Patent document cited in search report | | | Publication date (day/month/year) | Patent family member(s) | | | Publication date (day/month/year) |
|---|---|---|---|---|---|---|---|
| WO | 2011/105535 | A1 | 01 September 2011 | (Family: none) | | | |
| JP | 2010-77111 | A | 08 April 2010 | (Family: none) | | | |
| WO | 2020/188698 | A1 | 24 September 2020 | US | 2022/0142900 | A1 | |
| | | | | EP | 3943530 | A1 | |
| | | | | KR | 10-2021-0127741 | A | |
| | | | | CN | 113710729 | A | |
| | | | | TW | 202034896 | A | |
| WO | 2020/137017 | A1 | 02 July 2020 | CN | 113226277 | A | |
| | | | | KR | 10-2021-0107772 | A | |
| JP | 2014-28785 | A | 13 February 2014 | (Family: none) | | | |
| JP | 2007-291035 | A | 08 November 2007 | (Family: none) | | | |
| JP | 2020-109074 | A | 16 July 2020 | (Family: none) | | | |
| WO | 2012/133018 | A1 | 04 October 2012 | CN | 103442685 | A | |
| | | | | KR | 10-2014-0036158 | A | |
| | | | | HK | 1186687 | A | |
| | | | | TW | 201302228 | A | |
| WO | 2010/067037 | A1 | 17 June 2010 | JP | 2012-511556 | A | |
| | | | | US | 2012/0027825 | A1 | |
| | | | | EP | 2376218 | A1 | |
| | | | | FR | 2939699 | A | |
| | | | | CA | 2746706 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003146829 A
- WO 2011105535 A

- JP 6872068 B

**Non-patent literature cited in the description**

- *Oleo Science*, 2001, vol. 1 (6), 63-71

- International Cosmetic Ingredient Dictionary and Handbook. Personal Care Products Council, 2010